# EUROPEAN PATENT SPECIFICATION

(11) **EP 4 185 302 B1**
(45) Date of publication and mention of the grant of the patent: **03.09.2025**
(21) Application number: 21756028.3
(22) Date of filing: 22.06.2021
(51) Int. Cl.: A61K 31/728, A61K 36/58, A61K 36/752, A61P 15/02

(54) **COMPOSITION FOR THE PREVENTION AND THE TREATMENT OF VAGINAL DISEASES**
ZUSAMMENSETZUNG ZUR VORBEUGUNG UND BEHANDLUNG VON VAGINALERKRANKUNGEN
COMPOSITION POUR LA PRÉVENTION ET LE TRAITEMENT DE MALADIES VAGINALES

(30) Priority: 22.06.2020 IT 202000014911
(43) Date of publication of application: 31.05.2023
(73) Proprietor: Neilos S.r.l., 80063 Piano di Sorrento (NA) (IT)
(72) Inventor: DI MAIO, Umberto, 80065 Sant'Angelo (NA) (IT)
(74) Representative: Di Giovine, Paolo
(86) International application number: PCT/IB2021/055498
(87) International publication number: WO 2021/260546

(56) References cited:
- WO-A1-2015/136096
- WO-A1-2019/155389
- WO-A2-2007/132950
- WO-A2-2013/093941
- US-A1- 2011 206 789
- US-A1- 2013 108 599
- US-A1- 2019 275 099
- ZARETZKY FRANCA R ET AL: "Sulfated polyanions block Chlamydia trachomatis infection of cervix-derived human epithelia", BIOSIS, BIOSCIENCES INFORMATION SERVICE, PHILADELPHIA, PA, US, 1 January 1995 (1995-01-01), XP002802142
- SANTIAGO PALACIOS ET AL: "Beneficial effects of a -based vaginal gel on cervical epithelization, vaginal microbiota and vaginal health: a pilot study in asymptomatic women", BMC WOMEN'S HEALTH, BIOMED CENTRAL LTD, LONDON, UK, vol. 17, no. 1, 16 March 2017 (2017-03-16), pages 1 - 6, XP021242818, DOI: 10.1186/S12905-017-0374-2
- DATABASE BIOSIS [online] BIOSCIENCES INFORMATION SERVICE, PHILADELPHIA, PA, US; 1995, ZARETZKY FRANCA R ET AL: "Sulfated polyanions block Chlamydia trachomatis infection of cervix-derived human epithelia", XP002802142, Database accession no. PREV199598453864
- INFECTION AND IMMUNITY, vol. 63, no. 9, 1995, pages 3520 - 3526, ISSN: 0019-9567

## Description

The present invention relates to a composition of substances, comprising a combination of hyaluronic acid present as sodium hyaluronate with an average molecular weight comprised from 50 kDa to 3000 kDa, an extract of Citrus limon and an extract of Azadirachta indica, more commonly referred to as Neem. Said composition is effective in the prevention and/or treatment of vaginal diseases for use as a structural and protective re-epithelization agent against external attacks of bacterial, fungal, viral, mechanical and chemical nature.

Furthermore, forming an object of the present invention is a composition comprising a combination or mixture comprising or, alternatively, consisting of: (a) hyaluronic acid present as sodium hyaluronate with an average molecular weight comprised from 50 kDa to 3000 kDa, (b) an extract of Citrus limon, and (c) an extract of Azadirachta indica (Neem); for use in a method of preventive or curative treatment of vaginal diseases, wherein (a) the hyaluronic acid is present as sodium hyaluronate with an average molecular weight comprised from 50 kDa to 3000 kDa, and wherein (b) the extract of *Citrus limon* is a fruit extract of *Citrus limon*
Furthermore, forming an object of the present invention is a composition comprising a combination or mixture comprising or, alternatively, consisting of: (a) hyaluronic acid present as sodium hyaluronate with an average molecular weight comprised from 50 kDa to 3000 kDa, (b) an extract of Citrus limon, and (c) an extract of Azadirachta Indica (Neem), wherein (a) the hyaluronic acid is present as sodium hyaluronate and wherein (b) the extract of *Citrus limon* is a fruit extract of *Citrus limon.*

### Background of the invention

### The vaginal mucosa

The problems that frequently affect the female intimate mucosa bring with them unequivocal and clearly recognisable symptoms: irritations, burning sensations, itching, reddening, discomfort, often accompanied by abnormal vaginal discharge. In most cases, this is due to the proliferation of microorganisms of fungal, bacterial and, although with a lower incidence, viral or parasitic origin.

In the case of fungal diseases, the main culprits are fungi of the genus Candida (involved in 70-75% of acute vaginitis, with a tendency often to relapse), while the main accused blamed for bacterial diseases are Gardnerella vaginalis and the bacteria coming from the faecal reservoir (Escherichia coli, Streptococco faecalis, etc.).

More rare, but still possible, diseases of viral origin (genital herpes and human papillomavirus are the most significant examples) or diseases of parasitic origin (for example caused by Tricomonas vaginalis).

In order to understand how to prevent and treat these diseases it is first of all good to understand that the predisposition to intimate diseases is linked not only to the lack of the equilibrium of the ecosystem of the vaginal mucosa (change in pH, low presence of lactobacilli, etc.) but is also closely linked (especially in the case of Candida, but not only) to the imbalance of the intestinal microbial flora. The chosen approach must therefore act at both levels, ensuring effectiveness and, at the same time, absolute innocuity.

The mucous membranes of the intimate parts and the intestinal mucosa are closely linked, even if anatomically separated: a sort of network, which originates at the intestinal level, actually directly and indirectly affects the healthy or unhealthy conditions of the intimate parts. In this, a crucial role is played by the microbial flora, whose balance ("eubiosis" condition), not only directly hinders the invasion by possible pathogens, but also facilitates the correct functionality of the immune system present under the mucous membranes. Balance of physiological flora therefore also means balance (and therefore promptness of response) of the immune system, which is dedicated to local (and general) defence; vice versa, intestinal imbalance ("dysbiosis") results in a propensity to (intestinal and intimate) diseases. Lastly, the anatomical vicinity between the anarectal mucosa and the urogenital mucosa makes it clear that the more the physiological intestinal flora is in dysbiosis, the easier it is for the microorganisms present in large amounts in the faeces (Escherichia coli, Klebsiella, Streptococco faecalis, etc.) to contaminate the vulvar and vaginal mucous membranes. Although potentially pathogenic, some of these microorganisms are not capable of carrying out their damaging action precisely because they are numerically limited by lactobacilli and the immune system.

### Vaginal diseases

However, it may happen that the "good" vaginal flora alters, both qualitatively and quantitatively, and be overwhelmed by a mixed flora, rich in "bad" germs. Such condition, commonly known as bacterial vaginosis, occurs - for example - when taking antibiotics and immunosuppressants, when suffering from diabetes or severe psychophysical stress. This condition is manifested by the onset of a bad vaginal odour, sometimes associated with white-greyish and milky discharge. Generally, the symptoms that manifest themselves in the various vaginitis derive from the process of imbalance that causes inflammation, therefore the mucous membranes become more sensitive, the pH changes, the local immune system is fully active, the part in question is more sprayed with blood and malodorous compounds are formed.

The first-choice therapy in the treatment of vaginal infections is antibiotic.

Unfortunately, antibiotics have several side effects, the most important of which being the destruction of the vaginal and intestinal bacterial flora, with an increase in the possibility of bacterial and fungal relapse. Amoxicillin is the most active antibiotic on Gram positive bacteria (Gram+), including lactobacilli, which therefore succumb leaving freedom of action to vaginal colonisation by Candida, E. Coli and other Gram-negative bacteria (Gram-) (Herthelius et al 1989). The choice of antibiotic varies depending on the type of pathogen involved, in the case of aerobic vaginitis kanamycin is administered through the vaginal route while tetracyclines are used in the case of Mycoplasma Hominus infection. Candida with antimycotics (azoles and polyenes), Herpes Simplex Virus with antivirals.

WO 2015/136096 A1 discloses a topical composition comprising an extract of Coriolus versicolor for use in the prevention and/or in the treatment of a vaginal or cervical disorder caused by an infectious agent, through the vaginal or cervical route.

WO 2007/132950 A2 discloses a vaginal gel comprising lemon juice in liquid form and in powdered form. The safety, efficacy and acceptability of lemon juice as contraceptive (spermicide) and virucide (microbicide) against HIV was tested in such document.

SANTIAGO PALACIOS ET AL (" Beneficial effects of a -based vaginal gel on cervical epithelization, vaginal microbiota and vaginal health: a pilot study in asymptomatic women", BMC WOMEN'S HEALTH, BIOMED CENTRAL LTD, LONDON, UK, vol. 17, no. 1, pages 1-6) is a study aimed at verifying the effects of a treatment with a vaginal gel based on niosomes containing hyaluronic acid, b-glucan, alpha-glucan oligosaccharide, Coriolus versicolor, centella asiatica, Azadirachta indica and Aloe vera on vaginal microbiota, cervical epithelization and vaginal health.

WO 2019/155389 A1 discloses a muco-adhesive and bioadhesive aqueous composition for the treatment of diseases of the mucous membranes of the nasal cavity, of the oral cavity, of the respiratory and gastrointestinal tracts, of the eye, of the vagina, and of the bladder.

WO 2013/093941 A2 discloses a composition comprising probiotic lactobacilli strains for improved vaginal health.

DATABASE BIOSIS [Online] BIOSCIENCES INFORMATION SERVICE, PHILADELPHIA, PA, US; 1995, ZARETZKY FRANCA R ET AL: "Sulfated polyanions block Chlamydia trachomatis infection of cervix-derived human epithelia", is a study aimed at demonstrating that Chlamydia trachomatis infection can be blocked by some sulfated polysaccharides (carrageenan, pentosan polysulfate, fucoidan, and dextran sulfate) and glycosaminoglycans (heparin, heparan sulphate and dextran sulfate), and not by other glycosaminoglycans (chondroitin sulfate A or C, keratan sulfate and hyaluronic acid).

US 2013/108599 A1 discloses a herbal vaginal composition for the treatment of vaginal infections comprising: coconut oil as a base ingredient, shea butter, neem oil, lavender oil, vitamin E, lactobacillus, green tea, tea tree, ylang ylang, sweet orange, myrrh, incense, and clove oil.

US 2019/275099 A1 discloses a treatment regimen for an intravaginal yeast infection comprising an application device consisting of a drug to be inserted into a vagina. The drug comprises a liquid compound consisting of 55%±5% coconut oil, 20%±2.5% tea tree oil or neem oil, and 25%±2.5% apple cider vinegar.

It is clear that in order to prevent and resolve intimate diseases, it is always necessary to act not only at local level, where the symptom manifests itself, but also at intestinal level, in both cases counting on the efficacy and innocuity in the prevention and/or treatment of vaginal diseases. This is possible due to the use of specific formulations based on plant extracts.

In the composition subject of the present invention the synergistic action occurs between hyaluronic acid (HA) and/or a salt thereof, an extract of Citrus limon and an extract of Azadirachta indica, more commonly known as Neem. Said composition is effective in the prevention and/or treatment of vaginal diseases for use as a structural and protective re-epithelization agent against external attacks of bacterial, fungal, viral, mechanical and chemical nature. The scope of the invention is defined by the claims. Any references in the description to methods of treatment refer to the compounds, pharmaceutical compositions and medicaments of the present invention for use in a method for treatment of the human (or animal) body by therapy (or for diagnosis).

### GLOSSARY

The terms used in the present description are as generally understood by the man skilled in the art, unless otherwise indicated.

In the context of the present description, the expression "extract" is used to indicate any product related to a drug or plant matter including all products derived from mechanical treatments (pulverisation, crushing, mixing and/or other methods) and/or from extract-based treatments (solvent extraction, distillation, and/or other specific methods) conducted on said drug or plant matter.

### Hyaluronic acid

Hyaluronic acid is a molecule produced by the body to protect itself from infections, to increase plasticity and hydration of tissues. Hyaluronic acid is a high molecular weight polysaccharide of the group of glycosaminoglycans, formed by a linear chain, consisting of N-acetyl glucosamine and glucuronic acid disaccharide units. This substance is naturally produced by our body to hydrate and protect tissues and it is one of the main components of connective tissues on which it performs, together with collagen and elastin, a major structural function, conferring to the skin its particular properties of resistance, plasticity, turgor and density.

Its chemical-physical characteristics allow it to keep the skin elastic and to confer turgidity to the tissues. It is widely distributed in the various regions of our organism, we find it in fact in the vitreous body, in the umbilical cord, in the synovial fluid, in the joints, in the skin, in the heart, in the vessels, in the nails and in the urogenital system. It is synthesised on the inner surface of the plasma cell membrane by enzymes called hyaluronic acid synthase (HAS1, HAS2 and HAS3) and degraded through a mechanism of hydrolysis by proteins called hyaluronidase, whose crucial role is to cleave the molecules of high molecular weight hyaluronic acid so as to allow the carrying out of the multiple functions thereof.

As a matter of fact, hyaluronic acid is capable of covalently binding to its specific receptors CD44, RHAAM and ICAM, through which it interacts with the various cellular processes and it carries out the following activities: repair, lubricating, barrier, proliferation of damaged tissue and regeneration. Hyaluronic acid is continuously produced in our organism and it allows the maintenance of normal tissue functions. However, the production thereof decreases as age progresses or in the presence of particular conditions, leading to the onset of problems such as skin ageing, the loss of compactness and elasticity of the skin, the onset of joint problems and vaginal diseases. Specifically due to its characteristics, it is used in various fields: it is actually widely used in joint therapies, in dermatology, in case of skin lesions, sores or burns, but also in gynaecology for the women's health and in urology both in men and women. Vaginal diseases may be due to various factors: as a matter of fact, morphological changes, such as ectopias, metaplasia, hyperkeratosis or due to the lack of oestrogens, as occurs during the menopause and also physical and chemical treatments, such as DTC (diathermocoagulation), laser therapies, radiotherapy, are predisposing factors to the possible attack by pathogenic agents. This leads to change in the composition and structure of the epithelium which manifests itself with the typical signs of vaginal diseases characterised by symptoms such as: reddening, dryness, itching, burning sensation, dyspareunia, erythema, oedema, ulceration, bleeding. There is therefore a loss of integrity of the vaginal tissue and hyaluronic acid is one of the substances mainly involved in this process.

Actually, various studies argue that by interacting with the receptors thereof, hyaluronic acid is capable of repairing tissue damage and restoring the normal physiological conditions of the vaginal mucosa, intervening in all stages of the repair of the epithelium: creating a structure for supporting cell anchorage; inhibiting the flow of water on the cell surface; modulating the inflammatory response through the release of cytokines such as IL-6, TNF and IL-1 b; inducing angiogenesis, migration and proliferation of keratinocytes and fibroblasts during the proliferation and re-epithelization.

Supplementation with hyaluronic acid through the local or oral route may be a valuable aid for the resolution of vaginal diseases and the symptoms related thereto. However, in order to be effective, hyaluronic acid must have a very low molecular weight; scientific studies show that only a hyaluronic acid with a molecular weight less than 300 kDa is capable of penetrating into the tissues and repairing tissue damage, whereas larger molecules only act superficially.

Furthermore, further studies have shown that hyaluronic acid molecules with very low molecular weight can overcome the intestinal barrier and thus act systemically by distributing themselves in the various regions of the organism that need it. Unlike current therapies which provide for the use of lubricant gels which act only superficially and for a short period of time, or hormone replacement therapies, which could lead to side effects, hyaluronic acid carries out its repair action in depth, in a more durable manner over time and it is fully biocompatible. Due to its ability to bind water molecules and other substances, HA gives rise to protective gels, particularly useful both for dermal and intra-articular application and, by participating in the formation of collagen and connective tissue, it confers hydration and safety to the skin.

Furthermore, hyaluronic acid is considered an anti-bruise molecule, with a lubricating, healing and anti inflammatory action, given that it prevents damage due to physical stress and it hinders the uncontrolled spread of particular substances, such as bacteria and infectious agents.

Costantino et al. evaluated the efficacy and safety of a vaginal formulation in ovules containing HA, vitamin A and vitamin E. The results obtained confirmed a good safety profile of the product even after a long period of treatment of collagen and connective tissue. It protects the organism from viruses and bacteria and at the same time it increases the plasticity of the tissues while guaranteeing optimal skin hydration, conferring elasticity and softness to the skin. HA also has a significant healing and anti inflammatory activity (Brown et al., 2005; Zaleski et al., 2006; Favia et al. 2008) in women with vaginal atrophy. The study showed good efficacy on vaginal dryness and related symptoms starting from the first week of treatment and the absence of adverse effects in most patients (Costantino et al., 2008).

Hyaluronic acid may be used as an adjuvant in atrophic and dystrophic conditions, and in senile dryness due to estrogenic deficiency. Given that in menopause the vagina loses collagen and the ability to retain water, hyaluronic acid is used as an adjuvant in the processes of repairing astrophic and dystrophic conditions of the vaginal mucosa and in senile vaginal dryness due to oestrogen deficiency. In a clinical study, Morali et al. evaluated the efficacy of a gel formulation containing hyaluronic acid in 100 postmenopausal women with urogenital atrophy in order to evaluate the efficacy and safety thereof. The results showed a remarkable efficacy of the product on vaginal dryness and on all other symptoms and signs with a statistically significant reduction from the first week of treatment. In the study by Ekin et al., the administration of a formulation containing 5 mg hyaluronic acid to 21 postmenopausal women with atrophic vaginitis for 8 weeks resulted in a significant improvement in vaginal symptoms, epithelial atrophy, vaginal pH and vaginal maturation index (Ekin M. 2011). Hyaluronic acid forms a small part of the extracellular matrix (ECM), but it has the significant advantage of preserving the structure irrespective of the source and it is therefore non-allergenic. The chemical structure of hyaluronic acid appears to be preserved among the various living species, thus lowering its immunogenicity (Price RD. 2007). In the study by Morali et al., no treatment-related adverse events were reported by women. Therefore, the treatment can be considered effective and safe and an alternative to the treatment of genital atrophy in postmenopausal women, especially when hormonal therapy is not recommended (Morali G. 2006).

Hyaluronic acid has been widely used in cosmetic products due to its viscoelastic properties and its excellent biocompatibility. The application of hyaluronic acid on the skin leads to hydration and restoration of elasticity. Various studies on the chemical, physical-chemical properties and the physiological role of hyaluronic acid in men, together with the versatile properties such as biocompatibility, non-immunogenicity, biodegradability and viscoelasticity, have proven hyaluronic acid to be an ideal biomaterial for cosmetic, medical and pharmaceutical use (Brown MB. 2005).

The various activities (repair, lubrication, barrier, proliferation of damaged tissue and regeneration) of hyaluronic acid are therefore of considerable interest for the development of functional products in the treatment and in the prevention of vaginal diseases as a re-epithelization agent in damaged tissues, healing the micro-lesions and strengthening the base structure that becomes more resistant against external attacks (bacterial, fungal, viral, mechanical and chemical).

The remarkable properties of hyaluronic acid optimise the present formulation or composition, and they are assisted by the action of lemon and Neem plant. In the composition of the present invention the synergistic action occurs between hyaluronic acid and/or a salt thereof, an extract of Citrus limon and an extract of Azadhiracta indica, more preferably known as Neem.

### Citrus limon

Lemon (Citrus limon) isa fruit tree belonging to the family Rutaceae, and it originates from India and Indochina.

Lemon is a citrus fruit with multiple therapeutic properties. Lemon juice has purifying, antiseptic, disinfecting and bactericidal properties. The rind is antiseptic, while the essential oil is antipyretic, bactericidal and antiseptic. Lemon contains a good amount of vitamin C(ascorbic acid - up to 50 mg per 100 gr of edible part) and is therefore an excellent antioxidant against free radicals. It also contains mineral salts(in particular potassium: up to 140 mg per 100 gr of edible part) which make it a good daily supplement. Flavonoids, in particular rutin and hesperidin, a group of phytomolecules having an antioxidant and cholesterol-lowering action, are also present.

Although lemon juice has an acidic pH, due to the presence of citric acid, once metabolised it will perform an alkalinising action in the organism preventing kidney stones and uric acid build-up. Besides being used in the culinary field, lemon - in particular the juice thereof -is used as a skin detergent, given that it has marked antibacterial properties.

On the other hand, citroflavonoids contained in lemon have interesting therapeutic properties. More particularly, they have an anti-inflammatory activity and - affecting the vascular permeability - they are also capable of exerting a protective action against the blood capillaries. Furthermore, numerous studies have been carried out on the potential antitumor properties of limonene contained in lemon essential oil. These studies have shown that this molecule is capable of inhibiting in vitro the growth of certain types of tumour cells. Though the results obtained have been encouraging, new and more in-depth clinical studies are needed to demonstrate the actual effectiveness of limonene in combating tumour growth. These properties depend on the content of many vitamins and mineral salts. Though the most known component of this fruit is certainly vitamin C, whose important properties are universally known, also the percentage of water and citric acid is equally remarkable. Vitamin C may have additional roles in wound healing, for example by promoting keratinocyte differentiation and stimulating the formation of the epidermal barrier.

Besides vitamin C, lemon also contains vitamin B1 useful for the nervous system, vitamin B2 effective for growth as well as for nerve cell activity, and vitamin PP (niacin) also indispensable for the nervous system and the digestive system. Considered important among the mineral salts is the content of potassium, calcium and phosphorus, important for muscle contraction, nerve transmission and bone matrix. In the outer part of the fruit (epicarp) there are numerous essential oils, among which limonene, terpene, camphene, phellandrene and pinene, which have an antiseptic, bactericidal, anti-fungal, antipyretic, eupeptic, sedative action on the nervous system and stimulant action on the immune system. The bactericidal action of lemon peel is effective against cholera vibrion, meningococcus, staphylococci, gonococcus and numerous bacilli, including typhus, tuberculosis and diphtheria bacilli (Kobert, Cavel, Morel, Tecca, Sangiorgi, Catosi, Cadeac and Meunier). In a study by Hindi et al., the antimicrobial activity of lemon juice was shown to be higher than other plant extracts (leaves, roots, peels) against Gram positive, Gram negative and pathogenic yeasts. In particular, various isolates extracted from the lemon plant ( Citrus limon) and the lime plant ( Citrus limetta) were tested using the agar diffusion method. The lemon extracts showed a high antibacterial potential against 13 isolated bacteria out of 15 (including Candida a.), with the exception of S. epidermidis and P. aeruginosa which showed no susceptibility. Another study demonstrated the efficacy of vitamin C and silver citrate in the form of vaginal cream at acidic pH in the recurrence of bacterial vaginosis prophylaxis. Whereas bacterial vaginosis is a condition characterised by a depletion of lactobacilli, associated with the development of vaginal flora composed of aerobic, anaerobic and micro-aerophilic species, relapse can also be facilitated by inability to restore a dominant and protective lactobacillus flora in a non-optimal vaginal ecosystem, due to the persistence of a moderately high vaginal pH (4.5 to 4.6). Ascorbic acid performs an immediate acidifying action. In the aforementioned study, the direct microscopic examination of the vaginal discharge after about 2 weeks showed the presence of lactobacilli alone in 29 patients (72.5%), the increase in the percentage of lactobacilli with respect to the basic condition, and the corresponding decrease in the presence of pleomorphic bacteria. Furthermore, among its many properties, it facilitates cell reproduction and collagen formation, stimulating RNA protein synthesis. Last but not least, it is capable of enhancing cell-mediated immunity, activating chemotaxis and phagocytosis of neutrophils and macrophages (phagocytes).

### Azadirachta indica (Neem)

Neem (popular name of the goddess Neemari in India) is the tree of Azadirachta indica (Azad-Darakcht "Free Tree") which grows in the tropical and sub-tropical areas of Asia, Africa, America, Australia, belonging to the family Meliacee. Though the seeds from which the homonymous oil is obtained are the most used part of the plant due to their high concentration of therapeutic properties, also the other parts of the tree contain substances with medicinal effects. The three main active ingredients of the Neem plant are azadirachtin, margocin and catechin.

The entire field of action of Neem (for individual parts of the tree or mixtures thereof) was thoroughly analysed and a broad spectrum of properties were recognised: analgesic, antipyretic, anti inflammatory, antihistaminic, diuretic, hypoglycaemic, antimicrobial, spermicide, anti urinary disorders, anti internal and superficial burning sensations. Neem oil contains molecules capable of combatting viruses, bacteria, fungi, and it has moisturising, regenerating and restructuring properties and it is therefore effective, for example, on the most common dermatological diseases. The oil has a marked anti-inflammatory activity due to the presence of prostaglandin inhibitors. Neem is particularly effective against eczema and psoriasis, it moisturises and protects the skin while working to heal irritation, desquamation, lesions. Thanks to limonoids and catechins, potent inhibitors of mediators of acute inflammation, Neem is used to soothe pain from muscle strain, arthritis, rheumatism. The effect is equal to that obtained with products such as phenylbutazone and cortisone, also due to the anti inflation and oedemas reduction action. Neem oil has antiviral, antibacterial, antiparasitic, antiseptic and antifungal properties that make it suitable not only for health care but also to effectively promote the activity of our immune system.

According to a research published by the Institute of Biochemistry and Biotechnology of Kwame Nkrumah University of Science and Technology (KNUST) in Ghana, Neem is rich in essential fatty acids (EFAs), triglycerides, vitamin E and calcium. The EFAs contained therein are, in order of quantity present, oleic acid, linoleic acid, palmitic acid and stearic acid. Due to these fatty acids and vitamin E, Neem oil penetrates deeply into the skin where it repairs the tiny cracks caused by the strong dryness and it is therefore particularly useful on the skin that tends to lose liquids. Once absorbed the oil acts with a potent rejuvenating effect and helps to restore elasticity to the tissues; Vitamin E eliminates free radicals in the skin preventing the oxidation process that produces them and it can also be used to accelerate the healing of small wounds and to reduce scars. Neem contains anti-inflammatory substances called nimbidin and nimbin which relieve swelling and reddening and quercetin which helps the body respond to inflammation by inhibiting the production and release of irritants such as histamine. Neem shows an antimicrobial effect by inhibiting microbial growth. In several in vitro studies, Azadirachtin has shown antibacterial activity against Staphylococcus aureus and MRSA (methicillin-resistant staphylococcus aureus). Azadirachtin plays a role as a free radical scavenger as it is a rich source of antioxidants. The results of a study found that it is the roots of the plant that exhibit the greatest anti-radical activity. In a study to examine and evaluate the antimicrobial activity of the leaf and bark extracts of Azadirachta indica, ethanol and the aqueous extract of the leaves and barks of Neem were tested against Escherichia coli and Staphylococcus aureus which are known to be resistant to various antibiotics. The efficacy of the extracts was studied and determined by applying different concentrations on the two bacterial strains cultivated using the disc diffusion method. In this study, the in vitro effect of extracts from different Neem plants (leaf and bark) on Staphylococcus aureus and Escherichia Coli was examined. The susceptibility of the tested bacteria to both extracts was determined by measuring the diameter of the inhibition zones formed around the plates. The comparison conducted showed that, the materials of fresh extracts of Neem were the most effective as regards both Escherichia coli and Staphylococcus aureus. The results showed that the efficacy of the extracts depended on the concentration used, therefore the increase in the concentration of the extract increased the inhibition zone. Anti-inflammatory activity is performed by regulating pro-inflammatory enzymatic activities, including cyclooxygenases (COX) and lipoxygenases (LOX). Another study was conducted to evaluate the anti-inflammatory effect of Neem seed oil in albino rats using the carrageenan-induced oedema model. The results found that the oil inhibited the formation of carrageenan-induced oedema. The results found that the oil progressively inhibited the formation of oedema when the dose increased from 0.25 ml to 2 ml/kg body weight.

Neem oil has an antifungal activity, inhibiting Trichosporon (gastrointestinal tract infections) and Geotrichum (bronchial, lung and mucus membrane infections). The increased efficacy and safety (no side effect) of use against Candida albicans was observed.

In particular, the ability to inhibit 15 strains of the genus Candida in various extracts derived from the plant was evaluated by measuring the minimum inhibitory concentration (MIC). Flexanic and ethanolic extracts of seed oil showed the best activity in hindering fungal growth (13 strains out of 15). The ability of methanoic extracts of Neem leaves against various pathogens was tested in a recent study. Plate assays showed better inhibition against growth of Staphilococcus and Salmonella typhi unlike Bacillus subtilis and E.coli which were less susceptible to leaf extracts (50 mg/ml). In addition, Neem extracts (50mg/ml) were also effective against various several species of Trichoderma. In a study by Pandey et al., a herbal ointment containing Aloe Vera, turmeric and Neem was evaluated to test antibacterial and antifungal activity. The evaluation was conducted using the plate inhibition method and the dilution method by comparing the MIC values and/or the microbicidal activity. The study showed that the ointment containing the mixture of the three herbs showed a higher antifungal activity than the antibacterial activity, thus useful in the treatment of burns, wounds, mycoses and skin infections.

Forming an object of the present invention is a composition comprising a combination of hyaluronic acid present as sodium hyaluronate with an average molecular weight comprised from 50 kDa to 3000 kDa, an extract of Citrus limon and an extract of Azadirachta indica, more commonly known as Neem, having the characteristics as defined in the attached claims. Said composition is effective in prevention and/or treatment of vaginal diseases for use as a structural and protective re-epithelization agent against external attacks of bacterial, fungal, viral, mechanical and chemical nature.

Furthermore, forming an object of the present invention is a composition comprising a combination or mixture comprising or, alternatively, consisting of: (a) hyaluronic acid present as sodium hyaluronate with an average molecular weight comprised from 50 kDa to 3000 kDa, (b) an extract of Citrus limon, and (c) an extract of Azadirachta indica (Neem); for use in a method of preventive or curative treatment of vaginal diseases, having the characteristics as defined in the attached claims.

Furthermore, forming an object of the present invention is a composition comprising a combination or mixture comprising or, alternatively, consisting of: (a) hyaluronic acid present as sodium hyaluronate with an average molecular weight comprised from 50 kDa to 3000 kDa, (b) an extract of Citrus limon, and (c) an extract of Azadirachta Indica (Neem), having the characteristics as defined in the attached claims.

The hyaluronic acid sodium salt with an average molecular weight comprised from 50 kDa to 3000 kDa, (a) is present in an amount comprised from 0.01 to 60%, preferably in an amount comprised from 0.02% to 40% by weight, more preferably in an amount comprised from 0.05% to 30% by weight, even more preferably in an amount comprised from 0.05% to 2% by weight, with respect to the total weight of the composition.

The extract of Citrus limon (b) is preferably present in an amount comprised from 0.01% to 20% by weight, more preferably in an amount comprised from 0.05 % to 10% by weight, more preferably in an amount comprised from 0.1% to 3% by weight, even more preferably in an amount comprised from 0.5% to 1.5% by weight, with respect to the total weight of the composition.

The extract of Azadirachta indica (Neem) is preferably present in an amount comprised from 0.01% to 30 % by weight, preferably in an amount comprised from 0.1% to 20% by weight, more preferably in an amount comprised from 0.05% to 10%, even more preferably comprised from 0.1% to 6% by weight, further preferably comprised from 0.3% to 5% by weight, with respect to the total weight of the composition.

Preferably, said amounts refer to a daily dose per dose unit.

Hyaluronic acid (a) is present as sodium hyaluronate with ar average molecular weight comprised from 50 kDa to 3000 kDa.

The extract of Citrus Limon (b) is a fruit extract of Citrus limon fruits, more preferably an extract of the rind of said fruits
The extract of Azadirachta indica (Neem) (c) is preferably a seed and/or tree extract. More preferably, said extract of Azadirachta indica (c) comprises or, alternatively, consists of a Neem seed oil. Preferably, said Neem seed oil has an oleic acid titre comprised from 50% to 90%, preferably comprised from 60% to 80%, more preferably comprised from 65% to 75%, even more preferably comprised from 68% to 72%, for example 68%, 68.5%, 69%, 69.5%, 70%, 70.5%, 71%, 71.5% or 72%.

The dose form may be a pharmaceutical composition, or a dietary supplement composition, or a medical device composition or a food for special medical purposes including or comprising or, alternatively consisting of the aforementioned active ingredients mixed together.

The preferred route of administration is oral or topical. Said composition is therefore formulated for oral or topical use.

Said vaginal diseases are preferably of fungal, bacterial, viral and/or parasitic origin, more preferably they are of fungal and/or bacterial origin.

Even more preferably, said vaginal diseases of fungal and/or bacterial origin are caused by a fungus of the genus Candida, preferably a fungus of the species Candida albicans, and/or by a bacterium of the species Gardnerella vaginalis, Escherichia coli, or Streptococco faecalis.

Said composition is preferably for use as a structural and protective re-epithelization agent against external attacks of bacterial, fungal, viral, mechanical and chemical nature in a human or animal subject.

The following examples are given purely by way of illustration and they do not limit the scope of protection of the invention as defined in the attached claims. Changes or variations to the embodiments exemplified herein, obvious to the person skilled in the art, are encompassed by the attached claims.

### EXAMPLES

### Example 1

| Active ingredient | Daily dose per dose unit |
|---|---|
| (a) Sodium hyaluronate | 0.25% |
| (b) extract of *Citrus limon* (lemon) | 0.5% |
| (c) extract of *Azadirachta indica* (Neem oil) | 0.5% |

### Example 2

| Active ingredient | Daily dose per dose unit |
|---|---|
| (a) Hyaluronic acid | 0.05% |
| (b) extract of *Citrus limon* (lemon) | 0.5% |
| (c) extract of *Azadirachta indica* (Neem oil) | 0.5% |

### Example 3

| Active ingredient | Daily dose per dose unit |
|---|---|
| (a) Hyaluronic acid | 0.5% |
| (b) extract of *Citrus limon* (lemon) | 1.5% |
| (c) extract of *Azadirachta indica* (Neem oil) | 0.5% |

### Example 4

| Active ingredient | Daily dose per dose unit |
|---|---|
| (a) Hyaluronic acid | 0.3% |
| (b) extract of *Citrus limon* (lemon) | 1% |
| (c) extract of *Azadirachta indica* (Neem oil) | 5% |

### Example 5

| Active ingredient | Daily dose per dose unit |
|---|---|
| (a) Hyaluronic acid | 1% |
| (b) extract of *Citrus limon* (lemon) | 20% |
| (c) extract of *Azadirachta indica* (Neem oil) | 20% |

### Example 6

### EXPERIMENTAL PART

The synergistic action of the composition with respect to the individual active ingredients is evaluated through experimental methods in vitro and/or in vivo. The susceptibility of the cellular system to pro-inflammatory stimuli can be evaluated using immunoenzymatic assays (ELISA), by detecting the amount of cytokines involved in inflammatory processes and in particular the activity of bioactive compounds on the inhibition/activation response of cytokines (TNF-a, IL-1, IL-4, IL-6, IL-8, IL-10.IL-17, INF-Y, COMP), proinflammatory enzymes such as lipoxygenase (LOX) and cyclooxygenase (COX2) involved during inflammatory processes (e.g., LPS-induced) through Whole Blood Assay. Cell viability is one of the most widely used in vitro tests to assess the regenerative capacity of damaged tissues. The method of measuring cell viability is based on the reduction of resazurin (non-fluorescent) to resorufin (highly fluorescent) because there is a direct correlation between resazurin reduction and metabolic activity of viable cells. Cell cultures are prepared in complete medium and two concentrations of ethanol (for example 5% and 10%), used as positive control, are used. At the end of the treatment, a trypan blue count is conducted; the cell cultures are resuspended in previously heated resazurin (the reaction is temperature dependent). After an incubation period (about 60 minutes at controlled temperature), the medium is collected and read in fluorometer.

Other in vitro tests are acute inflammation models based on the use of UV rays or on the application of irritants. In particular, the different inhibitory effect of the active ingredients could be evaluated after the formation of oedema induced by the exposure of rats to UV rays.

Another method for evaluating the anti-inflammatory activity of the substances being tested is based on the cutaneous application of irritants (such as furfuryl ester). This test allows to evaluate the ability of the active agents to reduce induced erythema.

A further model for assessing inflammatory potential could be based on the study of the nuclear signalling pathway of the transcription factor kB which plays a primary role in the regulation of the inflammatory response. The ability of the active ingredients, alone or combined, to inhibit the activity of factor NF-kB could be evaluated through bioluminescence assays (luciferase assays for example). Antibacterial activity can be evaluated by determining the minimum inhibitory concentration (MIC) and minimum bactericidal concentration (MBC), using the broth dilution method. This test allows to detect the lowest concentration of a substance to be tested to inhibit the growth of various bacterial or fungal strains ( Escherichia coli, Streptococco faecalis, Staphilococcus aureus, Candida albicans etc.). At the end of the incubation period, the presence of bacterial or fungal growth is detected by means of the turbidity test. The test may be followed by the determination of the MCB (minimum bactericidal concentration); after dilution of the MIC, it is detected whether the culture shows growth or the test substance action is bactericidal.

Another widely used method is the disc diffusion technique and the plate diffusion technique. Suspensions of inocula of various bacterial or fungal strains on growth media are subcultured; paper discs in which the active ingredient to be tested is dissolved are subsequently added to the plates. This method allows to analyse the substances for their antifungal activity against the test fungi. In particular, during the plate diffusion test, after obtaining plates with colonies of Candida a. (for example), substances to be tested at a concentration greater than (10-fold) the minimum inhibitory concentration (MIC) are added to the obtained plates. The plates are placed at controlled temperature (around 30°C) and observed after about 48 hours. Inhibition of the growth of C. albicans is indicated by the appearance of a light halo at the point where the substances were added. The diameter of the inhibition zone of each substance was compared with the one obtained using a control with dimethyl sulfoxide (DMSO) and with the culture medium alone.

In order to demonstrate the anti-inflammatory and antibacterial efficacy of the composition according to the present invention, in vivo assays are also suitable to evaluate the properties of the individual substances and/or the synergistic activity of the mixture.

In particular, the inflammatory activity could be evaluated using the cotton pellet granuloma method. In this assay 2 cotton pellets are introduced into the abdomen of rats. The animals are divided into groups and subjected to daily administrations of the active ingredients under examination and of the carrier for a few consecutive days (in a period of 7 days, for example). At the end of the examination, the pellet is harvested with the granulomatous tissue formed. Then, the weights of the controls (largest cysts) are compared with those of the treated. This test evaluates the response of the active ingredients to induce the formation of smaller cysts and therefore show anti-inflammatory action.

In addition, an in vivo model based on vaginal infection in injured rats, in which regeneration of wounded tissues is tested, could be used to test the efficacy of the active ingredients of interest in cases of inflammation of the genital mucosa that also show tissue damage. In the in vivo test a wound is caused on the vaginal wall of rats and various bacterial strains are subsequently inoculated directly on the wound; the infection is repeated for 2 or more consecutive days. Subsequently, the active ingredients to be tested are injected into the vaginal tract and the produced vaginal discharge is harvested. The vaginal discharge is analysed and the inhibitory activity of the active agents of interest is tested by comparing the cell density analyses and the decrease in growth in plates of Lactobacilli. Lastly, an in vivo model for evaluating antibacterial activity is the one induced by stripping with adhesive tape. After shaving the back of anesthetised rats, stripping is conducted using an adhesive tape placed on an area of the hairless skin measuring 2.5 cm². Skin damage is defined as redness with no regular bleeding. Bacterial inoculation of a culture of Pseudomonas was subsequently carried out to cause infection. The rats were divided into 3 experimental groups: the control group with the wound on the skin without infection and treated with saline solution, the infected group treated with Pseudomonas and the group with the substance to be tested. Rats were anesthetised and sacrificed and after treatment about 300 mg of the wound were removed and homogenised. Lastly, a live bacteria plate count was conducted for CFU to compare the results between the study groups.

## Claims

1. A composition comprising a combination or mixture comprising or, alternatively, consisting of:
(a) hyaluronic acid and/or a salt thereof,
(b) an extract of *Citrus limon,* and
(c) an extract of *Azadirachta indica* (Neem);
for use in a method for the preventive or curative treatment of vaginal diseases,
wherein (a) the hyaluronic acid is present as sodium hyaluronate with an average molecular weight comprised from 50 kDa to 3000 kDa and wherein (b) the extract of *Citrus limon* is a fruit extract of *Citrus limon.*

2. The composition for use according to claim 1, wherein said vaginal diseases are of fungal, bacterial, viral and/or parasitic origin, preferably they are of fungal and/or bacterial origin.

3. The composition for use according to claim 2, wherein said vaginal diseases of fungal and/or bacterial origin are caused by a fungus of the genus *Candida,* preferably a fungus of the species Candida albicans, and/or by a bacterium of the species *Gardnerella vaginalis, Escherichia coli,* or *Streptococco faecalis.*

4. The composition for use according to any one of claims 1-3, wherein - with respect to the total weight of said composition (w/w):
(a) hyaluronic acid and/or a salt thereof is present in an amount comprised from 0.01% to 60% by weight, preferably in an amount comprised from 0.02% to 40% by weight, more preferably in an amount comprised from 0.05% to 30% by weight, even more preferably in an amount comprised from 0.05% to 2% by weight;
(b) the extract of *Citrus limon* is present in an amount comprised from 0.01% to 20% by weight, preferably in an amount comprised from 0.05% to 10% by weight, more preferably in an amount comprised from 0.1% to 3% by weight, even more preferably in an amount comprised from 0.5% to 1.5% by weight;
(c) the extract of *Azadirachta indica* (Neem) is present in an amount comprised from 0.01% to 30% by weight, more preferably in an amount comprised from 0.1% to 20% by weight, preferably comprised from 0.05% to 10% by weight, even more preferably comprised from 0.1% to 6% by weight, even more preferably comprised from 0.3% to 5% by weight.

5. The composition for use according to any one of claims 1-4, wherein (b) the extract of *Citrus limon* is an extract of the rind of said fruits, even more preferably it is an essential oil of the fruits thereof or of the rind of said fruits.

6. The composition for use according to any one of claims 1-5, wherein (c) the extract of *Azadirachta indica* (Neem) is a seed and/or tree extract, more preferably wherein said extract of *Azadirachta indica* (c) comprises or, alternatively, consists of a Neem seed oil, even more preferably wherein said Neem seed oil has an oleic acid titre comprised from 50% to 90%.

7. The composition for use according to any one of claims 1-6, wherein the composition is a medical device composition, a dietary supplement composition, a pharmaceutical composition, or a food for special medical purposes.

8. The composition for use according to any one of claims 1-7, wherein said composition is for use as a structural and protective reepithelialisation agent against external attacks of bacterial, fungal, viral, mechanical and chemical nature in a human or animal subject.

9. A composition comprising a combination or mixture comprising or, alternatively, consisting of:
(a) hyaluronic acid and/or a salt thereof,
(b) an extract of *Citrus limon*; and
(c) an extract of *Azadirachta indica* (Neem),
wherein (a) the hyaluronic acid is present as sodium hyaluronate with an average molecular weight comprised from 50 kDa to 3000 kDa and wherein (b) the extract of *Citrus limon* is a fruit extract of *Citrus limon.*

10. The composition according to claim 9, wherein - with respect to the total weight of said composition (w/w): (a) hyaluronic acid and/or a salt thereof is present in an amount comprised from 0.01% to 60% by weight, preferably in an amount comprised from 0.02% to 40% by weight, more preferably in an amount comprised from 0.05% to 30% by weight, even more preferably in an amount comprised from 0.05% to 2% by weight;
(b) the extract of *Citrus limon* is present in an amount comprised from 0.01% to 20% by weight, preferably in an amount comprised from 0.05% to 10% by weight, more preferably in an amount comprised from 0.1% to 3% by weight, even more preferably in an amount comprised from 0.5% to 1.5% by weight;
(c) the extract of *Azadirachta indica* (Neem) is present in an amount comprised from 0.01% to 30% by weight, preferably in an amount comprised from 0.1% to 20% by weight, more preferably comprised from 0.05% to 10% by weight, even more preferably comprised from 0.1% to 6% by weight, further preferably comprised from 0.3% to 5% by weight.

11. The composition according to claims 9 or 10, wherein (b) the extract of Citrus limon is an extract of the rind of said fruits, even more preferably it is an essential oil of the fruits thereof or of the rind of said fruits.

12. The composition according to any one of claims 9-11 , wherein (c) the extract of *Azadirachta indica* (Neem) is a seed and/or tree extract, preferably a seed extract, more preferably wherein said extract of Azadirachta indica (c) comprises or, alternatively, consists of a Neem seed oil, even more preferably wherein said Neem seed oil has an oleic acid titre comprised from 50% to 90%.

13. The composition according to any one of claims 9-12 or the composition for use according to any one of claims 1-8, wherein said composition is for oral or topical use.

## Patentansprüche

1. Zusammensetzung, umfassend eine Kombination oder Mischung, umfassend oder alternativ bestehend aus:
(a) Hyaluronsäure und/oder ein Salz davon,
(b) ein Extrakt aus *Citrus limon* und
(c) ein Extrakt aus *Azadirachta indica* (Neem);
zur Verwendung in einem Verfahren zur vorbeugenden oder heilenden Behandlung von vaginalen Krankheiten,
wobei (a) die Hyaluronsäure als Natriumhyaluronat mit einem durchschnittlichen Molekulargewicht von 50 kDa bis 3000 kDa vorliegt und wobei (b) der Extrakt aus *Citrus limon* ein Fruchtextrakt aus *Citrus limon* ist.

2. Zusammensetzung zur Verwendung nach Anspruch 1, wobei die vaginalen Krankheiten pilzlichen, bakteriellen, viralen und/oder parasitären Ursprungs sind, vorzugsweise sind sie pilzlichen und/oder bakteriellen Ursprungs.

3. Zusammensetzung zur Verwendung nach Anspruch 2, wobei die vaginalen Krankheiten pilzlichen und/oder bakteriellen Ursprungs durch einen Pilz der Gattung *Candida,* vorzugsweise einen Pilz der Spezies Candida albicans, und/oder durch ein Bakterium der Spezies *Gardnerella vaginalis, Escherichia coli* oder *Streptococco faecalis* verursacht werden.

4. Zusammensetzung zur Verwendung gemäß einem der Ansprüche 1 bis 3, wobei - bezogen auf das Gesamtgewicht der Zusammensetzung (Gew./Gew.):
(a) Hyaluronsäure und/oder ein Salz davon in einer Menge von 0,01 bis 60 Gew.-%, vorzugsweise in einer Menge von 0,02 bis 40 Gew.-%, noch bevorzugter in einer Menge von 0,05 bis 30 Gew.-%, noch mehr bevorzugt in einer Menge von 0,05 bis 2 Gew.-% vorhanden ist;
(b) der Extrakt aus *Citrus limon* in einer Menge von 0,01 bis 20 Gew.-%, vorzugsweise in einer Menge von 0,05 bis 10 Gew.-%, noch bevorzugter in einer Menge von 0,1 bis 3 Gew.-%, noch mehr bevorzugt in einer Menge von 0,5 bis 1,5 Gew.-% vorhanden ist;
(c) der Extrakt aus *Azadirachta indica* (Neem) in einer Menge von 0,01 bis 30 Gew.-%, vorzugsweise in einer Menge von 0,1 bis 20 Gew.-%, vorzugsweise von 0,05 bis 10 Gew.-%, noch bevorzugter von 0,1 bis 6 Gew.-% und noch bevorzugter von 0,3 bis 5 Gew.-% vorhanden ist.

5. Zusammensetzung zur Verwendung nach einem der Ansprüche 1 bis 4, wobei (b) der Extrakt aus *Citrus limon* ein Extrakt aus der Schale der Früchte ist, noch bevorzugter ist er ein ätherisches Öl der Früchte oder der Schale der Früchte.

6. Zusammensetzung zur Verwendung nach einem der Ansprüche 1 bis 5, wobei (c) der Extrakt aus *Azadirachta indica* (Neem) ein Samen- und/oder Baumextrakt ist, wobei der Extrakt aus *Azadirachta indica* (c) noch bevorzugter ein Neem-Samenöl umfasst oder alternativ aus einem Neem-Samenöl besteht, wobei das Neem-Samenöl noch bevorzugter einen Ölsäuregehalt von 50 % bis 90 % aufweist.

7. Zusammensetzung zur Verwendung nach einem der Ansprüche 1 bis 6, wobei die Zusammensetzung eine Zusammensetzung für medizinische Vorrichtungen, eine Nahrungsergänzungsmittelzusammensetzung, eine pharmazeutische Zusammensetzung oder ein Lebensmittel für besondere medizinische Zwecke ist.

8. Zusammensetzung zur Verwendung nach einem der Ansprüche 1 bis 7, wobei die Zusammensetzung zur Verwendung als strukturelles und schützendes Reepithelisierungsmittel gegen äußere Angriffe bakterieller, pilzlicher, viraler, mechanischer und chemischer Art bei einem menschlichen oder tierischen Subjekt bestimmt ist.

9. Zusammensetzung, umfassend eine Kombination oder Mischung, umfassend oder alternativ bestehend aus:
(a) Hyaluronsäure und/oder ein Salz davon,
(b) ein Extrakt aus *Citrus limon*; und
(c) ein Extrakt aus *Azadirachta indica* (Neem),
wobei (a) die Hyaluronsäure als Natriumhyaluronat mit einem durchschnittlichen Molekulargewicht von 50 kDa bis 3000 kDa vorliegt und wobei (b) der Extrakt aus *Citrus limon* ein Fruchtextrakt aus *Citrus limon* ist.

10. Zusammensetzung nach Anspruch 9, wobei - bezogen auf das Gesamtgewicht der Zusammensetzung (Gew./Gew.): (a) Hyaluronsäure und/oder ein Salz davon in einer Menge von 0,01 bis 60 Gew.-%, vorzugsweise in einer Menge von 0,02 bis 40 Gew.-%, noch bevorzugter in einer Menge von 0,05 bis 30 Gew.-%, noch mehr bevorzugt in einer Menge von 0,05 bis 2 Gew.-% vorhanden ist;
(b) der Extrakt aus *Citrus limon* in einer Menge von 0,01 bis 20 Gew.-%, vorzugsweise in einer Menge von 0,05 bis 10 Gew.-%, noch bevorzugter in einer Menge von 0,1 bis 3 Gew.-%, noch mehr bevorzugt in einer Menge von 0,5 bis 1,5 Gew.-% vorhanden ist;
(c) der Extrakt aus *Azadirachta indica* (Neem) in einer Menge von 0,01 bis 30 Gew.-%, vorzugsweise in einer Menge von 0,1 bis 20 Gew.-%, noch bevorzugter in einer Menge von 0,05 bis 10 Gew.-%, noch mehr bevorzugt in einer Menge von 0,1 bis 6 Gew.-% und ferner bevorzugt in einer Menge von 0,3 bis 5 Gew.-% vorhanden ist.

11. Zusammensetzung nach Anspruch 9 oder 10, wobei (b) der Extrakt aus Citrus limon ein Extrakt aus der Schale der Früchte ist, noch bevorzugter ein ätherisches Öl der Früchte oder der Schale der Früchte.

12. Zusammensetzung nach einem der Ansprüche 9 bis 11, wobei (c) der Extrakt aus *Azadirachta indica* (Neem) ein Samen- und/oder Baumextrakt, vorzugsweise ein Samenextrakt ist, vorzugsweise wobei der Extrakt aus Azadirachta indica (c) ein Neem-Samenöl umfasst oder alternativ aus einem Neem-Samenöl besteht, noch bevorzugter wobei das Neem-Samenöl einen Ölsäuregehalt von 50 % bis 90 % aufweist.

13. Zusammensetzung nach einem der Ansprüche 9 bis 12 oder Zusammensetzung zur Verwendung nach einem der Ansprüche 1 bis 8, wobei die Zusammensetzung zur oralen oder topischen Verwendung bestimmt ist.

## Revendications

1. Composition comprenant une combinaison ou un mélange comprenant ou, alternativement, constitué de :
(a) l'acide hyaluronique et/ou l'un de ses sels,
(b) un extrait de *Citrus limon,* et
(c) un extrait *d'Azadirachta indica* (margousier) ;
destinée à être utilisée dans un procédé pour le traitement préventif ou curatif des maladies vaginales,
dans laquelle (a) l'acide hyaluronique est présent sous forme d'hyaluronate de sodium avec une masse moléculaire moyenne allant de 50 kDa à 3000 kDa et dans laquelle (b) l'extrait de *Citrus limon* est un extrait de fruit de *Citrus limon.*

2. Composition destinée à être utilisée selon la revendication 1, dans laquelle lesdites maladies vaginales sont d'origine fongique, bactérienne, virale et/ou parasitaire, de préférence d'origine fongique et/ou bactérienne.

3. Composition destinée à être utilisée selon la revendication 2, dans laquelle lesdites maladies vaginales d'origine fongique et/ou bactérienne sont causées par un champignon du genre *Candida,* de préférence un champignon de l'espèce *Candida albicans,* et/ou par une bactérie de l'espèce *Gardnerella vaginalis, Escherichia coli,* ou *Streptococco faecalis.*

4. Composition destinée à être utilisée selon l'une quelconque des revendications 1 à 3, dans laquelle, par rapport au poids total de ladite composition (p/p) :
(a) l'acide hyaluronique et/ou l'un de ses sels sont présents en une quantité allant de 0,01 % à 60 % en poids, de préférence en une quantité allant de 0,02 % à 40 % en poids, plus préférablement en une quantité allant de 0,05 % à 30 % en poids, encore plus préférablement en une quantité allant de 0,05 % à 2 % en poids ;
(b) l'extrait de *Citrus limon* est présent en une quantité allant de 0,01 % à 20 % en poids, de préférence en une quantité allant de 0,05 % à 10 % en poids, plus préférablement en une quantité allant de 0,1 % à 3 % en poids, encore plus préférablement en une quantité allant de 0,5 % à 1,5 % en poids ;
(c) l'extrait *d'Azadirachta indica* (margousier) est présent en une quantité allant de 0,01 % à 30 % en poids, plus préférablement en une quantité allant de 0,1 % à 20 % en poids, de préférence allant de 0,05 % à 10 % en poids, encore plus préférablement allant de 0,1 % à 6 % en poids, encore plus préférablement allant de 0,3 % à 5 % en poids.

5. Composition destinée à être utilisée selon l'une quelconque des revendications 1 à 4, dans laquelle (b) l'extrait de *Citrus limon* est un extrait du zeste desdits fruits, encore plus préférablement une huile essentielle desdits fruits ou du zeste desdits fruits.

6. Composition destinée à être utilisée selon l'une quelconque des revendications 1 à 5, dans laquelle (c) l'extrait *d'Azadirachta indica* (margousier) est un extrait de graine et/ou d'arbre, plus préférablement dans laquelle ledit extrait *d'Azadirachta indica* (c) comprend ou, alternativement, est constitué d'une huile de graine de margousier, encore plus préférablement dans laquelle ladite huile de graine de margousier a un titrage d'acide oléique allant de 50 % à 90 %.

7. Composition destinée à être utilisée selon l'une quelconque des revendications 1 à 6, dans laquelle la composition est une composition pour dispositif médical, une composition pour complément alimentaire, une composition pharmaceutique ou un aliment destiné à des fins médicales spéciales.

8. Composition destinée à être utilisée selon l'une quelconque des revendications 1 à 7, dans laquelle ladite composition est destinée à être utilisée comme agent de réépithélialisation structurel et protecteur contre les attaques externes de nature bactérienne, fongique, virale, mécanique et chimique chez un sujet humain ou animal.

9. Composition comprenant une combinaison ou un mélange comprenant ou, alternativement, constitué de :
(a) l'acide hyaluronique et/ou l'un de ses sels,
(b) un extrait de *Citrus limon* ; et
(c) un extrait *d'Azadirachta indica* (margousier),
dans laquelle (a) l'acide hyaluronique est présent sous forme d'hyaluronate de sodium avec une masse moléculaire moyenne allant de 50 kDa à 3000 kDa et dans laquelle (b) l'extrait de *Citrus limon* est un extrait de fruit de *Citrus limon.*

10. Composition selon la revendication 9, dans laquelle, par rapport au poids total de ladite composition (p/p) : (a) l'acide hyaluronique et/ou l'un de ses sels sont présents en une quantité allant de 0,01 % à 60 % en poids, de préférence en une quantité allant de 0,02 % à 40 % en poids, plus préférablement en une quantité allant de 0,05 % à 30 % en poids, encore plus préférablement en une quantité allant de 0,05 % à 2 % en poids ;
(b) l'extrait de *Citrus limon* est présent en une quantité allant de 0,01 % à 20 % en poids, de préférence en une quantité allant de 0,05 % à 10 % en poids, plus préférablement en une quantité allant de 0,1 % à 3 % en poids, encore plus préférablement en une quantité allant de 0,5 % à 1,5 % en poids ;
(c) l'extrait *d'Azadirachta indica* (margousier) est présent en une quantité allant de 0,01 % à 30 % en poids, de préférence en une quantité allant de 0,1 % à 20 % en poids, plus préférablement allant de 0,05 % à 10 % en poids, encore plus préférablement allant de 0,1 % à 6 % en poids, encore plus préférablement allant de 0,3 % à 5 % en poids.

11. Composition selon la revendication 9 ou 10, dans laquelle (b) l'extrait de *Citrus limon* est un extrait du zeste desdits fruits, encore plus préférablement une huile essentielle desdits fruits ou du zeste desdits fruits.

12. Composition selon l'une quelconque des revendications 9 à 11, dans laquelle (c) l'extrait *d'Azadirachta indica* (margousier) est un extrait de graine et/ou d'arbre, de préférence un extrait de graine, plus préférablement dans laquelle ledit extrait *d'Azadirachta indica* (c) comprend ou, alternativement, est constitué d'une huile de graine de margousier, encore plus préférablement dans laquelle ladite huile de graine de margousier a un titrage d'acide oléique allant de 50 % à 90 %.

13. Composition selon l'une quelconque des revendications 9 à 12 ou composition destinée à être utilisée selon l'une quelconque des revendications 1 à 8, dans laquelle ladite composition est destinée à être utilisée par voie orale ou topique.
